# EUROPEAN PATENT APPLICATION

(11) **EP 4 838 206 A1**
(43) Date of publication of application: **11.11.2026**
(21) Application number: 25175072.5
(22) Date of filing: 08.05.2025
(51) Int. Cl.: G01N 21/78, G01N 21/31, G01N 31/22, G01N 33/18

(54) **A METHOD OF DETERMINING AN INTERFERENCE AND A CONCENTRATION OF A NITRATE IN AN AQUEOUS MEDIA AND A SYSTEM FOR PERFORMING THE METHOD**

(71) Applicant: Hach Lange GmbH, 14163 Berlin (DE)
(72) Inventor: RUDDE, Heinz, 14163 Berlin (DE); HAHN, Markus, 14163 Berlin (DE)
(74) Representative: terpatent PartGmbB

(57) **Abstract**

A method of determining an interference and a concentration of a nitrate in an aqueous media includes providing a first sample of the aqueous media which has the nitrate as nitrate ions, mixing the first sample with a reagent comprising 2,6-dimethylphenol so that the nitrate ions react with the 2,6-dimethylphenol so as to form 4-nitro-2,6-dimethylphenol in a mixed first sample, determining a first absorbance of the nitrate in the mixed first sample colorimetrically at a first wavelength, determining a second absorbance of the nitrate in the mixed first sample colorimetrically at a second wavelength, comparing the first absorbance with the second absorbance to determine whether or not the interference of nitrite and/or of chloride exists, and determining a nitrate concentration based on whether or not the interference of nitrite and/or of chloride exists.

## Description

The present invention provides a method of determining an interference and a concentration of a nitrate in an aqueous media and a system for performing the method.

The nitrate ion is an oxidative ion with the Molecular formula NO₃⁻ and a molecular mass of 62.0049 g/mol. Nitrate represents the most oxidized chemical form of nitrogen found in natural systems. All living systems require nitrogen to exist since nitrogen is used to build many essential components such as proteins, DNA, RNA and vitamins, as well as hormones and enzymes. Higher organisms such as animals cannot use simple forms of nitrogen, such as nitrate and ammonium, and are instead dependent on complex forms of nitrogen such as amino acids and nucleic acids.

While nitrogen is an essential building block for life, nitrogen in the form of nitrate can also be harmful. When nitrate is taken in by eating food and drinking water, nitrate is converted to nitrite. Nitrite then combines with hemoglobin to form methemoglobin. This process can lead to the Hypoxia (medical) in organ tissue and a dangerous condition called Methemoglobinemia. Methemoglobinemia in infants is known as blue baby syndrome. Infants are more susceptible to nitrate toxicity than older children or adults. While fatalities are rare, sub-acute methemoglobinemia can affect development. Chronic consumption of high levels of nitrate may also cause other health problems, such as cancer or may contribute to disturbing the growth and development of an embryo or fetus. This is because excess nitrite moves into the bloodstream where it binds strongly to blood hemoglobin and impairs the delivery of oxygen to the embryo or fetus. Elevated levels of nitrate also lead to a build-up of nitrite in the gastrointestinal tract by nitrate reducing bacteria. There have recently been reports of a link between nitrate levels in drinking water and bladder cancer in women. Blood and serum nitrate levels can also become elevated as the result of increased production of nitric oxide (NO). Nitric oxide is an unstable gaseous compound that readily diffuses into body fluids where it can be converted to nitrate, nitrite or S-nitrothiol. NO levels rise during heightened immune-response such as occurs during sepsis, organ failure or graft-rejection.

Some adults can be more susceptible to the effects of nitrate than others. The Cytochrome b5 reductase Enzyme may be under-produced or absent in certain people that have an inherited mutation. Such individuals are unable to break down methemoglobin as rapidly as those that have the enzyme, leading to increased circulating levels of methemoglobin with the result that their blood is not as oxygen-rich. Those with insufficient stomach acid may also be at risk. Such individuals may include, for example, vegetarians and vegans. The increased consumption of green, leafy vegetables that typically accompanies vegetarian and vegan diets may lead to increased nitrate intake. While nitrate exposure is most easily caused by drinking water, it can also be caused by eating vegetables with high levels of nitrate. The high levels of nitrate in plants may be caused, for example, by growth conditions such as reduced sunlight, the undersupply of the essential micronutrients molybdenum (Mo) and iron (Fe), or high concentrations of nitrate due to reduced assimilation of nitrate in the plant. High levels of nitrate fertilization also contribute to elevated levels of nitrate in the harvested plant. A wide variety of medical conditions, such as food allergies, asthma, hepatitis, and gallstones may be linked with low stomach acid; these individuals may also be highly sensitive to the effects of nitrate.

Nitrate does not, however, only affect humans. Other animals are also affected. Nitrate can reach such high levels in some freshwater or estuary systems close to land so as to potentially cause the death of fish. Nitrate levels over 30 ppm can inhibit growth, impair the immune system and cause stress in some aquatic species. Supplying a nitrogen-limited eco-system with high levels of nitrate can result in significant increases in the levels of phytoplankton (algae) and macrophytes (aquatic plants). This can pose a significant threat to fragile ecosystems. The recommended level of nitrates to avoid the propagation of algal blooms is between 0.1 to 1 mg/l.

Nitrate is a wide spread contaminant of ground and surface waters worldwide. The accumulation of nitrate in the environment is greatly impacted by runoff from the over-application of nitrogen fertilizers. Nitrate contamination can also occur from concentrated animal feeding operations and from poorly or untreated human sewage. Because nitrate is a naturally-occurring chemical that is left after the breakdown or decomposition of animal or human waste, water quality may also be affected if a high number of septic systems exist in a watershed. Septics leach down into ground water resources or aquifers and supply nearby bodies of water. Lakes that rely on ground water are often affected by nitrification through this process. Nitrate-containing wastes are also produced by many industrial processes including paper and munitions manufacturing. The burning of fossil fuels in power plants and cars, SUVs and all internal combustion engines results in the production of nitric acid and ammonia as air pollution.

Nitrate ion from fertilizers, sewage and manufacturing has reached high concentrations in water supplies throughout the world. The analytical control of nitrate concentrations in surface waters, especially those which serve as drinking water sources, is therefore regulated in most advanced countries. The United States Environmental Protection Agency (EPA) has, for example, established an enforceable regulation for nitrate, called a maximum contaminant level (MCL), at 10 mg/l or 10 ppm. The EPA thereby notes that infants below six months who drink water containing nitrate in excess of the maximum contaminant level could become seriously ill and, if untreated, may die.

Because the major environmental release of nitrate arises from its use in fertilizers, it is unlikely that the nitrate problem will disappear anytime soon. A continued need to monitor nitrates in finished drinking water, watersheds, industrial wastewater, private wells and estuaries exists. Nitrate contamination of source water will also continue to be relevant for industries that depend on water purity for manufacturing their products.

In addition to other well-known specific nitrate determination methods, such as ion chromatography or direct potentiometry (so-called NO₃ ISE or ion selective electrode), colorimetric nitrate determination methods currently serve as the "backbone" of nitrate analyses in water laboratories.

One of the current state of the art colorimetric methods is the LCK 339 nitrate kit provided by Hach Lange GmbH. The LCK 339 nitrate kit provides a highly accurate and reliable nitrate analysis for waste water, drinking water, raw water, surface water, soils, substrates and nutrient solutions in the range of 0.23-13.50 mg/l NO₃-N with a cuvette path length of 11 mm. The LCK 339 nitrate kit determines the concentration of nitrate based on the principal that nitrate ions in solutions containing high concentrated sulfuric and phosphoric acids react with 2,6-dimethylphenol to form 4-nitro-2,6-dimethylphenol, which can in turn be detected colorimetrically at a wavelength of 340 nm.

The LCK 339 nitrate kit itself improves on known state of the art analytical methods whereby the concentration of NO₃-N is colorimetrically determined using 2,6-dimethylphenol.

The colorimetric detection of nitrate levels using the aforementioned methods has several disadvantages. For example, analytical methods using 2,6-dimethylphenol are sensitive to side reactions in the presence of chlorides. This leads to low NO₃-N recoveries and places a cap on the detection limit. Samples containing chloride, calcium or nitrite salt can furthermore either not be analyzed or can only be analyzed in a limited fashion. Sea water, brackish water and/or water from municipal wastewater treatment plants having a high salt content therefore usually cannot be analyzed with the aforementioned methods. Nitrate in ultra-pure water and in drinking water having a nitrate concentration below the aforementioned detection limits can also either not be detected or detected only imprecisely using the aforementioned methods. Wavelengths of between about 340 nm and 370 nm are also susceptible to interference which negatively impact detection accuracy.

As stated above, the LCK 339 nitrate kit determines the concentration of nitrate based on the principal that nitrate ions in solutions containing high concentrated sulfuric and phosphoric acids react with 2,6-dimethylphenol to form 4-nitro-2,6-dimethylphenol, which can in turn be detected colorimetrically at a wavelength of 340 nm. In the absence of interference, a weak side reaction can be detected with a wavelength peak of 510 nm. Samples containing chloride and/or nitrite can, however, interfere with the determined concentration of nitrate at 340 nm. In the presence of chloride, a slightly stronger side reaction will be formed with a wavelength peak of 510 nm. In the presence of nitrite, a strong side reaction will be formed with a wavelength peak of 510 nm. In the presence of both chloride and nitrite, the chloride content is active as an inhibitor and will slightly suppress the nitrite side reaction depending of the concentration of chloride and nitrite. The impact of this interference is non-linear so that nitrite interferes strongly in the lower measuring range and less strongly in the higher measuring range.

Other colorimetric nitrate methods such as the Spectroquant^{®} cuvette test 114556 offered by Merck make use of a side reaction, the so-called Liebermann Nitroso Reaction (LNR), in the presence of chlorides to form an intense colored product. This reaction is mainly used for the colorimetric determination of phenols. The application of the LNR as a nitrate analytical method has the disadvantage that long reaction times of at least 30 minutes are necessary for the complete reduction of the nitrate to the nitroso compound if chloride is the sole reducing agent. Measuring earlier would yield imprecise and inaccurate results. Nitrate tests using the LNR generally have a measuring range limit of 0.1 to 3.0 mg NO₃-N mg/l using a 16 mm cuvette. The variation coefficient of this method is, however, double that of the variation coefficient of the aforementioned nitrate analysis methods using 2,6-dimethylphenol. The 16 mm thickness of the cuvette is also almost 1.5 times thicker than the 11 mm cuvette used in the 2,6-dimethylphenol analysis method. The preciseness of this analysis method will therefore necessarily be less than that of the 2,6-dimethylphenol analysis method.

Nitrate concentration can, for example, be indirectly determined by reducing nitrate to nitrite. The reducing agent for this method is usually either granulated and/or activated cadmium or hydrazine salts, both of which are, however, highly poisonous, such that their sale and disposal is prohibited or limited in many countries for environmental reasons. Following reduction, the nitrite ions are then reacted with an aromatic amine to form a diazonium salt in the presence of an acidic buffer. The diazonium salt in turn reacts with N-(1-naphthyl)-ethylene-diamine to form a red-violet azo dye which allows a semi-quantitative visual comparison with a color scale to occur. Test strips, color cubes and color disks based on this principle are available. While easy to use, test strips and color cubes only allow nitrate concentrations to be determined at certain, limited, concentrations, such as, for example, at 0, 1, 2, 5, 10, 20 and 50 ppm NO₃-N for the AquaChek^{®} test strips from Hach Lange GmbH. The easy to use color disks allow for a slightly more accurate NO₃-N concentration to be determined, but are also limited to an accuracy in the mg/l NO₃-N range. The accuracy of the color disk NI-11 from Hach Lange GmbH, for example, is 0-50 mg/l NO₃-N. A somewhat more precise evaluation of the absorption of the color test solution is also possible photometrically.

Nitrate concentration can also be accurately measured through complex high-end systems such as the NITRATAX^{®} family of probes or the GANIMEDE laboratory analysers offered by Hach Lange GmbH. The NITRATAX^{®} probes are specifically designed to constantly monitor the concentration of nitrate by direct immersion in activated sludge, wastewater and/or surface water. The NITRATAX^{®} plus sc model, for example, has a very good measuring range of 0.1-100 mg/l NO₃-N. The automated GANIMEDE N analyzer offered by Hach Lange GmbH can analyze for total nitrate concentration. The GANIMEDE N operates using reagents to digest unwanted by-products and to thereby provide a "pure" water sample for analysis. Direct UV measurement at approximately 210 nm versus a reference wavelength of approximately 228 nm, high-quality optics and a complex algorithm are used to calculate nitrate concentrations at a very good measuring range of 0.5-150 mg/l NO₃-N (after digestion).

An aspect of the present invention is to provide an improved colorimetric nitrate analysis method where the concentration of nitrate is based on the principal that nitrate ions in solutions containing high concentrated sulfuric and phosphoric acids react with 2,6-dimethylphenol to form 4-nitro-2,6-dimethylphenol, which can in turn be detected colorimetrically, which method can adjust the detected concentration of nitrate if chloride and/or nitrite interreference exists, in a simple manner.

In an embodiment, the present invention provides a method of determining an interference and a concentration of a nitrate in an aqueous media. The method includes providing a first sample of the aqueous media which comprises the nitrate as nitrate ions, mixing the first sample with a reagent comprising 2,6-dimethylphenol, wherein the nitrate ions react with the 2,6-dimethylphenol so as to form 4-nitro-2,6-dimethylphenol in a mixed first sample, determining a first absorbance of the nitrate in the mixed first sample colorimetrically at a first wavelength, determining a second absorbance of the nitrate in the mixed first sample colorimetrically at a second wavelength, comparing the first absorbance with the second absorbance to determine whether or not the interference of nitrite and/or of chloride exists, and determining a nitrate concentration based on whether or not the interference of nitrite and/or of chloride exists. Preferably, if no nitrite interference exists, the first absorbance is seen to be proportional to the nitrate concentration.

In a preferred embodiment of the present invention, the first wavelength can, for example, be from 300 nm to 385 nm, preferably from 320 nm to 360 nm, very preferably from 340 nm to 350 nm, and most preferably at approximately 345 nm. The first wavelength can be exactly at 345 nm.

In a preferred embodiment of the present invention, the second wavelength can, for example, be from 450 nm to 570 nm, for example, from 470 nm to 550 nm, preferably from 490 nm to 530 nm, very preferably from 500 nm to 520 nm, most preferably at approximately 510 nm. The second wavelength can be exactly at 510 nm.

In a preferred embodiment of the present invention, the determining of the first absorbance and the determining of the second absorbance can be performed simultaneously or quasi-simultaneously to guarantee coherence.

In a preferred embodiment of the present invention, the comparing of the first absorbance with the second absorbance to determine whether or not the interference of nitrite and/or of chloride exists is provided by comparing the first absorbance with the second absorbance by means of or in a database; and/or comparing a ratio of the first absorbance to the second absorbance by means of or in the database.

In a preferred embodiment of the present invention, the determining of the nitrate concentration based on whether or not the interference of nitrite and/or of chloride exists comprises using the first absorbance of the nitrate in the mixed first sample to determine the nitrate concentration if no interference of nitrite and/or of chloride is determined to exist. If the interference of nitrite and/or of chloride is determined to exist, the method alternatively comprises determining an initial nitrate concentration based on the first absorbance, and adjusting the initial nitrate concentration determined based on the first absorbance via the database and/or via the ratio of the first absorbance to the second absorbance in the database to determine the nitrate concentration.

In a preferred embodiment of the present invention, the method can also include determining whether or not the interference of chloride and/or of nitrite is significant. If an interference is present, but is not deemed significant, then, as above, the method can use the first absorbance of the nitrate in the mixed first sample to determine the nitrate concentration as if no interference of nitrite and/or of chloride is determined to exist. If the interference of chloride and/or of nitrite is deemed significant, than, as above, the method alternatively comprises determining an initial nitrate concentration based on the first absorbance, and adjusting the initial nitrate concentration determined based on the first absorbance via the database and/or via the ratio of the first absorbance to the second absorbance in the database to determine the nitrate concentration.

Whether an interference is deemed significant will depend on the circumstances. As an example, a skilled person might have performed a wastewater analysis where historically 1 mg/l NO₃-N were measured without any measurable chloride or nitrite being present. The first absorption and second absorption measurements of the wastewater samples were historically 0,173 and 0,042 respectively, with the factor F (i.e., the ratio of the first absorbance to the second absorbance) being 4,12. If the skilled person would then measure first absorption and second absorption measurements of 0,350 and 0,547, respectively, with the factor F being 0,64, a significant interference clearly exists. A significant interference can generally be deemed to exist if the measured nitrate concentration deviates from the expected and/or actual nitrate concentration by 0.5-50 %, preferably 5-20 %, very preferably 10-15 %. Significance can alternatively simply be based on the factor F. A significant interference can generally be deemed to exist if the factor F calculated based on the first absorption and the second absorption deviates from the expected or historically obtained factor F by more than 35 %, preferably by more than 20 %, very preferably by more than 10 %, most preferably by more than 5 %. In the above case, the factor F measured (i.e., 0,64) deviated from the expected and/or historical factor F (i.e., 4,12) by (4,12-0,64)/4,12 •100 = 84 %. It is noted that if the factor F is higher (for example, 4,26), that the percentage can be calculated in the same way as (4,12-4,26)/4,12 •100 = - 3,4 % or 3,4 %.

In a preferred embodiment of the present invention, the adjusting of the initial nitrate concentration determined based on the first absorbance via the database and/or via the ratio of the first absorbance to the second absorbance in the database to determine the nitrate concentration is performed via a trained learning algorithm.

The trained learning algorithm can, for example, be an artificial neural network trained by a backpropagation method or a pre-trained convolutional neural network. It is generally known that machine learning is able to recognize patterns in signals. The trained learning algorithm can therefore be based on known machine learning methods, such as "Decision Tree Learning", "k-Nearest Neighbor", "Linear Regression", Logistic Regression", "Winnow", "LASSO", "Ridge-Regression", "ARIMA", "Perceptron", "Artificial Neural Networks", "Deep Learning", "Naive Bayes", "Bayesian Network", "Support Vector Machines", "Boosting", "Reinforcement Learning", "Markov Chain", "Hidden Markov Model", or other state of the art machine learning methods. Many of these known machine learning methods, including Perceptron, Winnow and Logistic Regression, have online versions capable of continuously learning from data. By utilizing such online learning methods, the quality of the adjusting of the initial nitrate concentration can continually be improved.

In a preferred embodiment of the present invention, the determining of the nitrate concentration based on whether or not the interference exists comprises using the first absorbance of the nitrate in the mixed first sample to determine the nitrate concentration if no interference of nitrite and/or of chloride is determined to exist. If the interference of nitrite and/or of chloride is determined to exist, the method alternatively comprises providing a second sample of the aqueous media which comprises the nitrate as nitrate ions, adjusting the second sample to mitigate the interference, mixing the second sample with 2,6-dimethylphenol, wherein the nitrate ions react with the 2,6-dimethylphenol so as to form 4-nitro-2,6-dimethylphenol in a mixed second sample, determining an absorbance of the nitrate in the mixed second sample colorimetrically at the first wavelength, and using the absorbance of the nitrate in the mixed second sample to determine the nitrate concentration.

In a preferred embodiment of the present invention, the adjusting of the second sample to mitigate the interference comprises at least one of adding amidosulfonic acid (H₂NSO₃H) to the second sample, and/or adding silver sulfate (Ag₂SO₄) to the second sample, and/or diluting the second sample by calculating a dilution factor, and/or adding a standard addition concentration to the second sample, and/or confirming a chloride concentration in the second sample. If the chloride concentration of a nitrite containing sample is known, the influence of the interference on the nitrate result can be better estimated.

The present invention also provides a system for determining an interference and a concentration of a nitrate The system essentially comprises a cuvette, a photometer, and a computer. The cuvette is configured to have a sample of an aqueous media which comprises the nitrate and a reagent comprising 2,6-dimethylphenol be mixed therein so as to provide a mixed sample. The photometer comprises a light source and a light detector. The photometer is configured to determine a first absorbance of the nitrate in the mixed sample colorimetrically at a first wavelength, and to determine a second absorbance of the nitrate in the mixed sample colorimetrically at a second wavelength. The computer comprises a monitor and a database. The database comprises comparisons of first absorbance values with respective second absorbance values and ratios of the first absorbance values to the respective second absorbance values. The computer is operatively linked to the photometer. The system is configured to perform the method as recited above.

The present invention also provides a test kit for determining a concentration of a nitrate in a sample, wherein the test kit uses the method as described above.

The present invention is described in greater detail below on the basis of embodiments and of the drawings in which:
Fig. 1 shows an example of absorbance measurements of a nitrate sample (bottom) and of a nitrate sample which has been spiked with 10 ppm of nitrite (top);
Fig. 2 shows an example of absorbance measurements of various nitrate standards, some of which have been spiked with chloride; no nitrite was here added;
Fig. 3 shows an example of absorbance measurements of various nitrate standards, some of which have been spiked with nitrite; no chloride was here added;
Fig. 4 shows an example of absorbance measurements of various nitrate samples, each of which have been spiked with chloride and/or additionally with nitrite;
Fig. 5 shows an example of absorbance measurements of various nitrate samples, each of which have been spiked with chloride and/or additionally with nitrite; the amount of NO₃-N is here higher than in the example of the absorbance measurements shown in Fig. 4;
Fig. 6 shows a flow chart of a method of determining a concentration of a nitrate in an aqueous media according to the prior art;
Fig. 7 shows a flow chart of a method of determining an interference and a concentration of a nitrate in an aqueous media according to the present invention; and
Fig. 8 shows a system which uses the method of determining an interference and a concentration of a nitrate in an aqueous media according to the present invention.

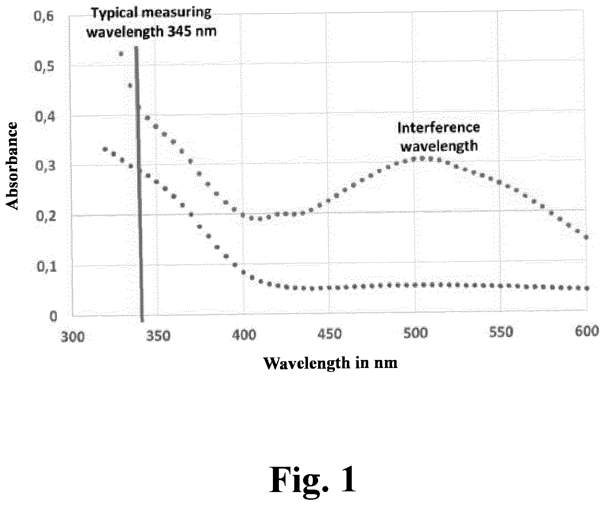

Fig. 1 shows an example of absorbance spectrum measurements E100, E110 of a nitrate sample without nitrite (bottom) and of a nitrate sample which has been spiked with 10 ppm of nitrite (top). Fig. 1 shows that the interference of nitrite will increase the absorbance at 345 nm and falsify the result. Fig. 1 also shows that the interference can best be monitored in the range of approximately 510 nm. The side reaction wavelength in the range of 510 nm can be monitored well even via a visual evaluation.

Further evidence of how chloride and/or nitrite interferes with absorbance measurements is provided in the data set forth in Table 1 below:

**Table 1**

| **Point** | **NO₃-N** / **NO₂⁻** / **Cl⁻in mg/l** | **NO₃-N Nominal Value in mg/l** | **NO₃-N Measured in mg/l** | **Deviation in %** |
|---|---|---|---|---|
| 1 | 1 / 0 / 0 | 1 | 1,03 | 3 |
| 2 | 1 / 10 / 0 | 1 | 1,78 | -78 |
| 3 | 1 / 0 / 400 | 1 | 0,97 | -3 |
| 4 | 1 / 10 / 400 | 1 | 1,53 | -53 |

Table 1 shows example results of the cuvette LCK 339 nitrate test kit provided by Hach Lange GmbH with various interferences of nitrite and/or chloride. The measurement range of the cuvette LCK 339 nitrate test kit is 0,23 to 13,5 mg/l NO₃-N with typical applications including wastewater, surface water, and drinking water. Table 1 shows that both nitrite and chloride interfere with a correct measured result of NO₃-N. Table 1 makes clear that even a small amount of nitrite (i.e., 10 mg/l) results in a significant deviation of the NO₃-N measured.

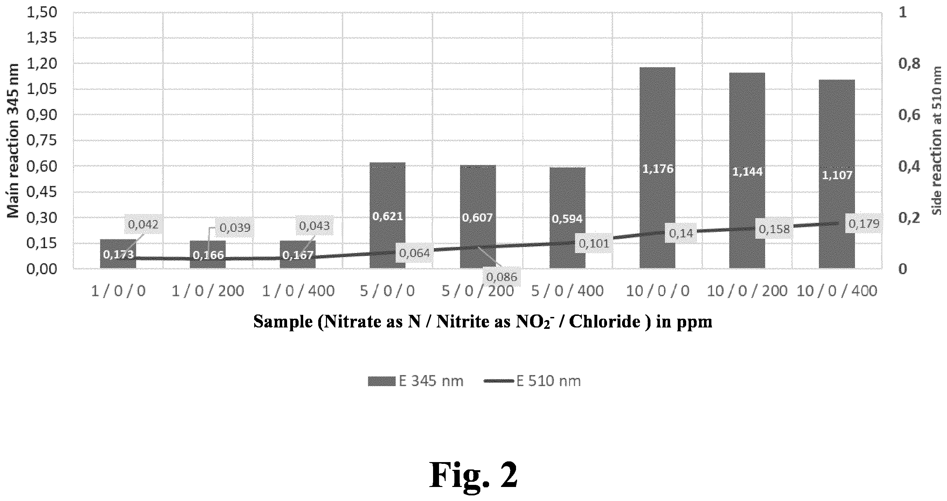

Fig. 2 shows an example of absorbance measurements of various nitrate standards, some of which have been spiked with chloride. No nitrite was here added. The data of Fig. 2 is plotted below in Table 2:

**Table 2**

| **Sample NO₃-N** / **NO₂⁻** / **Cl⁻in mg/l** | **Expected Result NO₃-N in mg/l** | **Result (based on interference) in mg/l** | **Deviation in %** | **Factor F** |
|---|---|---|---|---|
| 1 / 0 / 0 | 1,00 | 1,03 | 3 | 4,12 |
| 1 / 0 / 200 | 1,00 | 0.97 | -3 | 4,26 |
| 1 / 0 / 400 | 1,00 | 0,97 | -3 | 3,88 |
| 5 / 0 / 0 | 5,00 | 4,95 | -1 | 9,70 |
| 5 / 0 / 200 | 5,00 | 4,85 | -3 | 7,06 |
| 5 / 0 / 400 | 5,00 | 4,74 | -5 | 5,88 |
| 10 / 0 / 0 | 10,00 | 9,88 | -1 | 8,40 |
| 10 / 0 / 200 | 10,00 | 9,59 | -4 | 7,24 |
| 10 / 0 / 400 | 10,00 | 9,26 | -7 | 6,78 |

Table 2 shows that a slightly increasing absorbance at 510 nm here indicates a risk of interference. Chloride is a "weak" interference and leads to results that lie slightly below the target values. Fig. 2 also allows typical factors F to be calculated. For example, a 1 mg/l NO₃-N standard with 200 mg/l chloride has a factor F of (E 345 nm)/(E 510 nm) = 0,166/0,039 = 4,26.

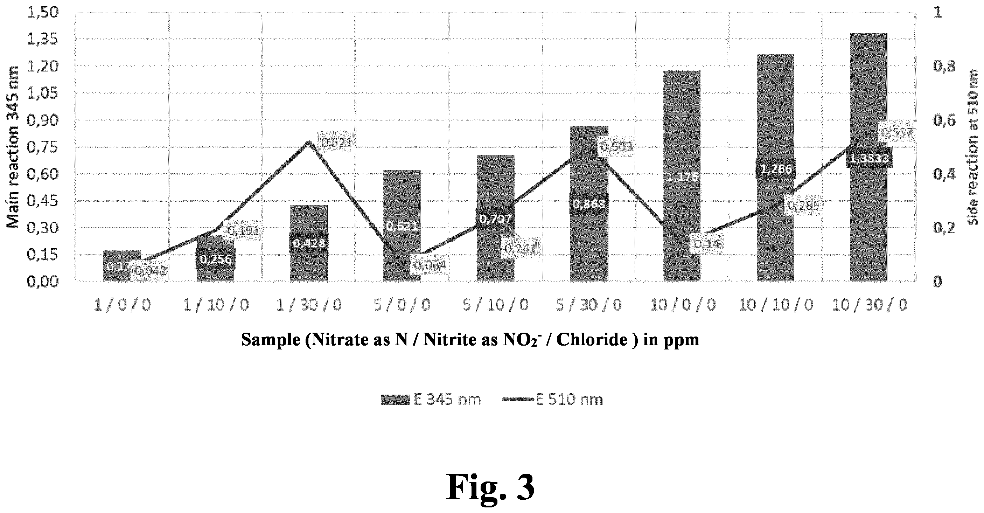

Fig. 3 shows an example of absorbance measurements of various nitrate standards, some of which have been spiked with nitrite. No chloride was here added. The data of Fig. 3 is plotted below in Table 3:

**Table 3**

| **Sample NO₃-N** / **NO₂⁻** / **Cl⁻ in mg/l** | **Expected Result NO₃-N in mg/l** | **Result (based on interference) in mg/l** | **Deviation in** % | **Factor F** |
|---|---|---|---|---|
| 1 / 0 / 0 | 1,00 | 1,03 | 3 | 4,05 |
| 1 / 10 / 0 | 1,00 | 1,78 | 78 | 1,34 |
| 1 / 30 / 0 | 1,00 | 3,33 | 233 | 0,82 |
| 5 / 0 / 0 | 5,00 | 4,95 | -1 | 9,70 |
| 5 / 10 / 0 | 5,00 | 5,78 | 16 | 2,93 |
| 5 / 30 / 0 | 5,00 | 7,19 | 44 | 1,73 |
| 10 / 0 / 0 | 10,00 | 9,88 | -1 | 8,40 |
| 10 / 10 / 0 | 10,00 | 10,70 | 7 | 4,44 |
| 10 / 30 / 0 | 10,00 | 11,70 | 17 | 2,48 |

Table 3 shows examples of nitrate standards with and without nitrite. A slightly increasing absorbance at 510 nm here indicates a risk of interference. Nitrite is a "strong" interference and generally leads to results that lie higher than the target values. Fig. 3 also allows typical factors F to be calculated. For example, a 1 mg/l NO3-N standard with 10 mg/l nitrite has a factor F of (E 345 nm)/(E 510 nm) = 0,256/0,191 = 1,34.

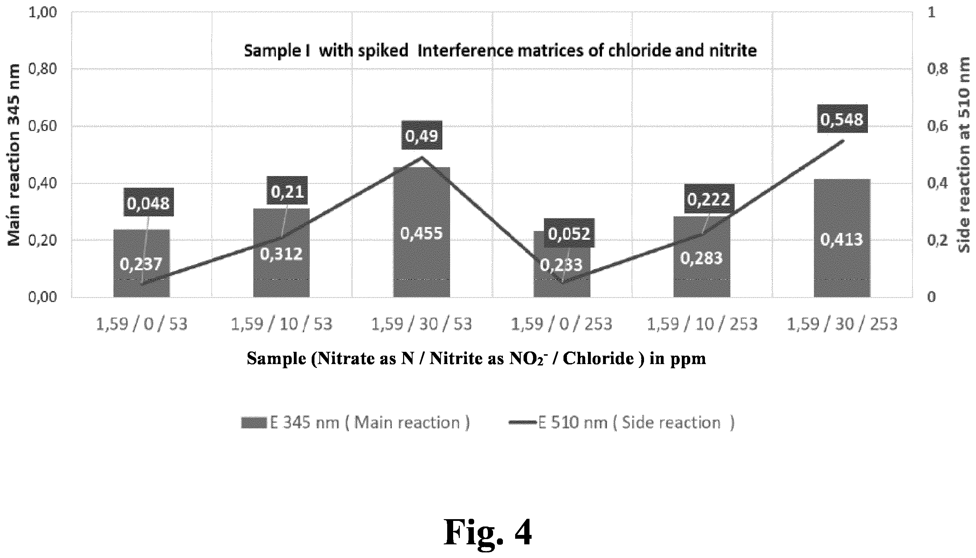

Fig. 4 shows an example of absorbance measurements of various nitrate samples, each of which have been spiked with chloride and/or additionally with nitrite. The data of Fig. 4 is plotted below in Table 4:

**Table 4**

| **Sample NO₃-N** / **NO₂⁻** / **Cl⁻in mg/l** | **Expected Result NO₃-N in mg/l** | **Result (based on interference) in mg/l** | **Deviation in** % | **Factor F** |
|---|---|---|---|---|
| 1,59 / 0 / 0 | 1,59 | 1,59 | 0 | 4,94 |
| 1,59 / 10 / 0 | 1,59 | 2,30 | 45 | 1,49 |
| 1,59 / 30 / 0 | 1,59 | 3,59 | 126 | 0,93 |
| 1,59 / 0 / 0 | 1,59 | 1,57 | -1 | 4,48 |
| 1,59 / 10 / 0 | 1,59 | 2,02 | 27 | 1,27 |
| 1,59 / 30 / 0 | 1,59 | 3,20 | 101 | 0,75 |

Table 4 shows examples of samples each of which have been spiked with chloride and/or additionally with nitrite. A differing and increasing absorbance at 510 nm here indicates a risk of interference. All demonstrated spiked samples lead to a false result. Fig. 4 also allows typical factors F to be calculated. For example, a 1,59 mg/l NO₃-N standard with 253 mg/l chloride has a factor F of (E 345 nm)/(E 510 nm) = 0,233/0,052 = 4,48; a 1,59 mg/l NO₃-N standard with 253 mg/l chloride and 10 mg/l nitrite has a factor F of (E 345 nm)/(E 510 nm) = 0,283/0,222 = 1,27.

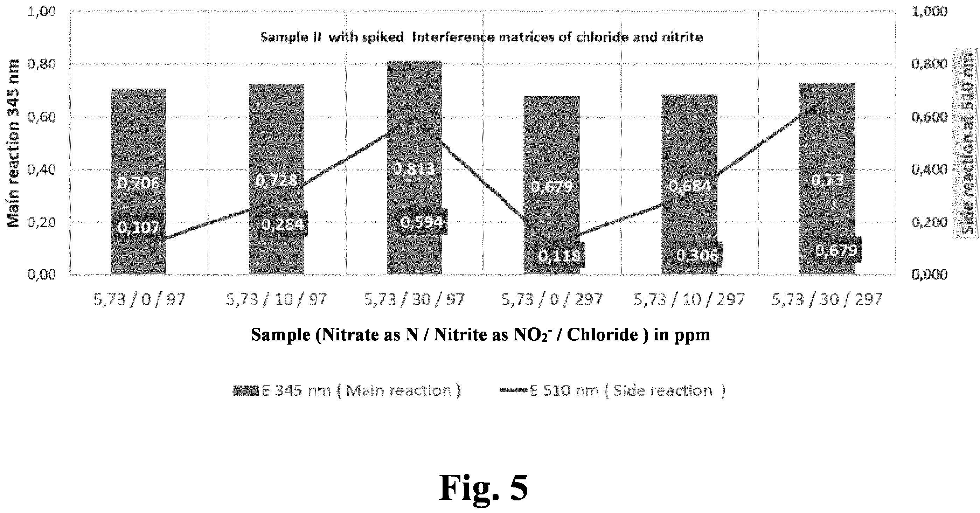

Fig. 5 shows an example of absorbance measurements of various nitrate samples, each of which have been spiked with chloride and/or additionally with nitrite. The amount of NO₃-N is higher in Fig. 5 than in the example of the absorbance measurements shown in Fig. 4. The data of Fig. 5 is plotted below in Table 5:

**Table 5**

| **Sample NO₃-N** / **NO₂⁻** / **Cl⁻ in mg/l** | **Expected Result NO₃-N in mg/l** | **Result (based on interference) in mg/l** | **Deviation in %** | **Factor F** |
|---|---|---|---|---|
| 5,73 / 0 / 97 | 5,73 | 5,73 | 0 | 6,60 |
| 5,73 / 10 / 97 | 5,73 | 5,96 | 4 | 2,56 |
| 5,73 / 30 / 97 | 5,73 | 6,70 | 17 | 1,37 |
| 5,73 / 0 / 297 | 5,73 | 5,50 | -4 | 5,75 |
| 5,73 / 10 / 297 | 5,73 | 5,51 | -4 | 2,24 |
| 5,73 / 30 / 297 | 5,73 | 5,98 | -4 | 1,08 |

Table 5 shows examples of samples each of which have been spiked with chloride and/or additionally with nitrite. A differing and increasing absorbance at 510 nm here indicates a risk of interference. Fig. 5 also allows typical factors F to be calculated. For example, a 5,73 mg/l NO₃-N standard with 297 mg/l chloride and 10 mg/l nitrite has a factor F of (E 345 nm)/(E 510 nm) = 0,684/0,306= 2,24. Table 5 shows that not all examples lead to a false result, i.e., 5,73 mg/l NO₃-N and 97 mg/l chloride yields the 5,73 expected result.

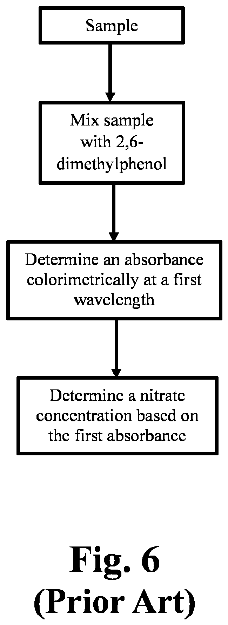

Fig. 6 shows a method of determining a concentration of a nitrate in an aqueous media according to the prior art. This prior art method provides a sample of the aqueous media which comprises the nitrate as nitrate ions. The sample would then be mixed with a reagent comprising 2,6-dimethylphenol, wherein the nitrate ions would react with the 2,6-dimethylphenol so as to form 4-nitro-2,6-dimethylphenol in a mixed first sample. An absorbance would then be determined colorimetrically at a wavelength of, for example, 345 nm. A nitrate concentration would then be determined based on the absorbance measured.

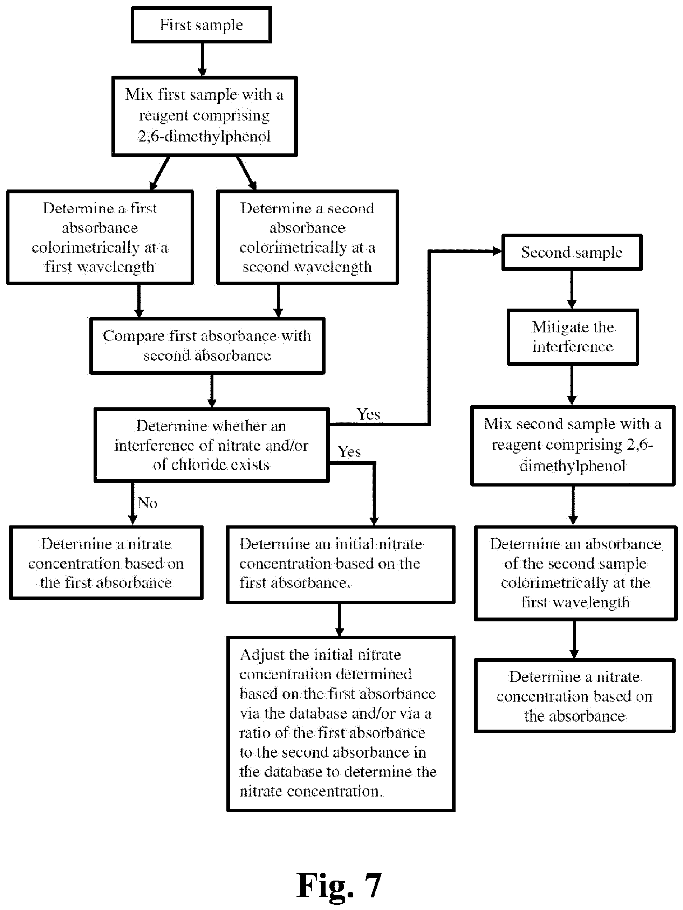

Fig. 7 shows a flow chart of the method according to the present invention. The method of the present invention provides a first sample of an aqueous media which comprises the nitrate as nitrate ions. As in the prior art, the first sample is mixed with a reagent comprising 2,6-dimethylphenol, wherein the nitrate ions react with the 2,6-dimethylphenol so as to form 4-nitro-2,6-dimethylphenol in a mixed first sample. In contrast to the prior art, the method of the present invention determines a first absorbance of the nitrate in the mixed first sample colorimetrically at a first wavelength, and a second absorbance of the nitrate in the mixed first sample colorimetrically at a second wavelength. The first absorbance is then compared with the second absorbance. Based on this comparison, it is then determined whether or not an interference of nitrite and/or of chloride exists.

The comparison is performed, for example by comparing the first absorbance with the second absorbance in a database, and/or comparing a ratio of the first absorbance to the second absorbance in the database. A sample database is set forth below as Table 6:
Table 6 shows an example of a "database point" for 1 mg/l NO₃-N at different interference levels of chloride and nitrate as measured at a main wavelength of 345 nm and at a second interference wavelength of 510 nm. Table 6 also shows the factor R (E 345 nm)/(E 510 nm) of each database point.

**Table 6**

| **1 mg/l NO₃-N** | **0 mg/l Cl⁻** | **Factor F** | **200 mg/l Cl⁻** | **Factor F** | **400 mg/l Cl⁻** | **Factor F** |
|---|---|---|---|---|---|---|
| 0 mg/l NO₂⁻ | 0,173 / 0,042 | 4,12 | 0,166 / 0,039 | 4,26 | 0,167 / 0,043 | 3,88 |
| 10 mg/l NO₂⁻ | 0,256 / 0,191 | 1,34 | 0,240 / 0,225 | 1,07 | 0,266 / 0,208 | 1,09 |
| 30 mg/l NO₂⁻ | 0,428 / 0,521 | 0,82 | 0,373 / 0,470 | 0,79 | 0,350 / 0,547 | 0,64 |

The database would ideally have much more data than the database points set forth above. The database would ideally have as many data points for a first absorbance, a second absorbance and factors F relating to the ratio thereof as possible. It is conceivable that any individual or entity performing the method of the present invention would input additional database points into the database to thereby continually improve and refine the method of the present invention for future measurements. As an example, a skilled person might be operating in a wastewater environment where a concentration of nitrate is usually around 1 mg/l with no or negligible chloride or nitrite interference. The expected first absorbance, second absorbance, and factor F values would thus be 0,173 / 0,042 and 4,12. If the skilled person, instead of obtaining these values, instead obtained first absorbance, second absorbance, and factor F values of 0,240 / 0,225 and 1,07, the skilled person would thus immediately conclude that interference of chloride and/or of nitrite exists. The skilled person might also immediately conclude, based on the database, that 100 mg/l chloride and 10 mg/l NO₂ exists in the sample.

A skilled person who obtained first absorbance, second absorbance, and factor F values of 0,173 / 0,042 and 4,12 would conclude that no interference exists and would thus determine a nitrate concentration based on the first absorbance. A skilled person obtaining first absorbance, second absorbance, and factor F values of 0,240 / 0,225 and 1,07 would in contrast conclude that interference does exist. The skilled person would thus provide a second sample of the aqueous media which comprises the nitrate as nitrate ions and adjust the second sample to mitigate the interference. This mitigation could involve adding amidosulfonic acid (H₂NSO₃H) to the second sample in order to remove nitrite. The skilled person alternatively or additionally add silver sulfate (Ag₂SO₄) to the second sample to remove chloride. The skilled person could alternatively or additionally dilute the sample by a dilution factor if the nitrate concentration lies outside of test parameters; the skilled person would in this case need to undertake a new interference determination. The skilled person could alternatively or additionally add a known standard addition concentration of nitrite and/or chloride to the second sample. The skilled person could alternatively or additionally confirm a chloride and/or a nitrite concentration in the second sample.

Once the interference has been mitigated, the skilled person would again mix the second sample with 2,6-dimethylphenol, wherein the nitrate ions react with the 2,6-dimethylphenol so as to form 4-nitro-2,6-dimethylphenol in a mixed second sample. The skilled person would then determine an absorbance of the nitrate in the mixed second sample colorimetrically at the first wavelength and subsequently use the absorbance of the nitrate in the mixed second sample to determine the nitrate concentration.

The skilled person who has confidence that a nitrate level must fall within a generally narrow range could alternatively simply adjust the initial nitrate concentration determined based on the first absorbance via the database and/or via the ratio of the first absorbance to the second absorbance in the database to determine the nitrate concentration. Based on Table 6, a skilled person obtaining first absorbance, second absorbance, and factor F values of 0,173 / 0,042 and 4,12 would simply conclude that the nitrate concentration was 1 mg/l NO₃-N.

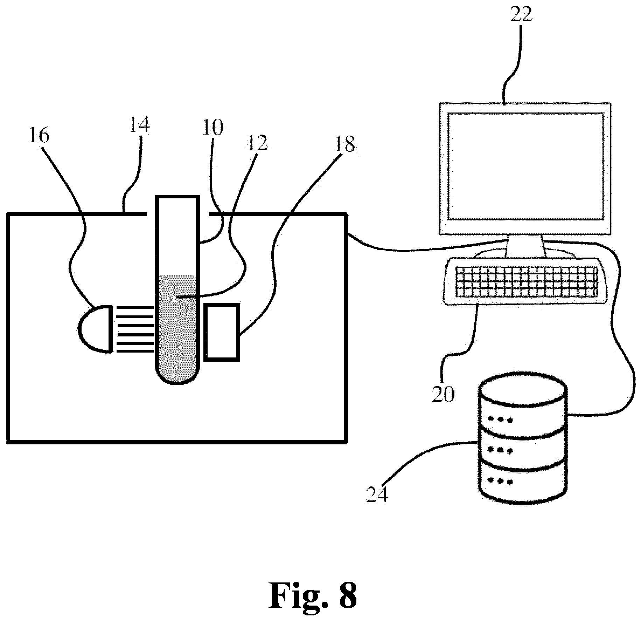

Fig. 8 shows a system for determining an interference and a concentration of a nitrate according to the present invention. A cuvette 10 is here configured to have a sample of an aqueous media which comprises the nitrate and a reagent comprising 2,6-dimethylphenol be mixed therein so as to provide a mixed sample 12. The nitrate ions thereby react with the 2,6-dimethylphenol so as to form 4-nitro-2,6-dimethylphenol. The cuvette 10 is placed into a photometer 14 comprising a light source 16 and a light detector 18. The photometer 14 is configured to determine a first absorbance of the nitrate in the mixed sample colorimetrically at a first wavelength, and to determine a second absorbance of the nitrate in the mixed sample colorimetrically at a second wavelength. The first wavelength is preferably approximately 345 nm, while the second wavelength is preferably approximately 510 nm. The system further comprises a computer 20 which comprises a monitor 22 and a database storage 24. The database storage 22 comprises comparisons of first absorbance values with respective second absorbance values and ratios of the first absorbance values to the respective second absorbance values. The computer 20 is operatively linked to the photometer 14. The system is configured to perform the method as recited above.

### LIST OF REFERENCE NUMERALS

- 10: Cuvette
- 12: Mixed sample
- 14: Photometer
- 16: Light source
- 18: Light detector
- 20: Computer
- 22: Monitor
- 24: Database

## Claims

1. A method of determining an interference and a concentration of a nitrate in an aqueous media, the method comprising:
providing a first sample of the aqueous media which comprises the nitrate as nitrate ions;
mixing the first sample with a reagent comprising 2,6-dimethylphenol, wherein the nitrate ions react with the 2,6-dimethylphenol so as to form 4-nitro-2,6-dimethylphenol in a mixed first sample;
determining a first absorbance of the nitrate in the mixed first sample colorimetrically at a first wavelength;
determining a second absorbance of the nitrate in the mixed first sample colorimetrically at a second wavelength;
comparing the first absorbance with the second absorbance to determine whether or not the interference of nitrite and/or of chloride exists; and
determining a nitrate concentration based on whether or not the interference of nitrite and/or of chloride exists.

2. The method as recited in claim 1, wherein,
the first wavelength is from 300 nm to 385 nm, preferably 345 nm, and
the second wavelength is from 450 nm to 570 nm, preferably 510 nm.

3. The method as recited in at least one of claims 1 and 2, wherein the determining of the first absorbance and the determining of the second absorbance is performed simultaneously or non-simultaneously.

4. The method as recited in at least one of claims 1-3, wherein the comparing of the first absorbance with the second absorbance to determine whether or not the interference of nitrite and/or of chloride exists is provided by:
comparing the first absorbance with the second absorbance in a database; and/or
comparing a ratio of the first absorbance to the second absorbance in the database.

5. The method as recited in claim 4, wherein the determining of the nitrate concentration based on whether or not the interference of nitrite and/or of chloride exists comprises:
using the first absorbance of the nitrate in the mixed first sample to determine the nitrate concentration if no interference of nitrite and/or of chloride is determined to exist, or,
if the interference of nitrite and/or of chloride is determined to exist,
determining an initial nitrate concentration based on the first absorbance, and
adjusting the initial nitrate concentration determined based on the first absorbance via the database and/or via the ratio of the first absorbance to the second absorbance in the database to determine the nitrate concentration.

6. The method as recited in claim 5, wherein the adjusting the initial nitrate concentration determined based on the first absorbance via the database and/or via the ratio of the first absorbance to the second absorbance in the database to determine the nitrate concentration is performed via a trained learning algorithm.

7. The method as recited in at least one of claims 1-4, wherein the determining the nitrate concentration based on whether or not the interference exists comprises:
using the first absorbance of the nitrate in the mixed first sample to determine the nitrate concentration if no interference of nitrite and/or of chloride is determined to exist, or,
if the interference of nitrite and/or of chloride is determined to exist,
providing a second sample of the aqueous media which comprises the nitrate as nitrate ions;
adjusting the second sample to mitigate the interference; mixing the second sample with 2,6-dimethylphenol, wherein the nitrate ions react with the 2,6-dimethylphenol so as to form 4-nitro-2,6-dimethylphenol in a mixed second sample;
determining an absorbance of the nitrate in the mixed second sample colorimetrically at the first wavelength; and
using the absorbance of the nitrate in the mixed second sample to determine the nitrate concentration.

8. The method as recited in claim 5, wherein the adjusting of the second sample to mitigate the interference comprises at least one of:
adding amidosulfonic acid (H₂NSO₃H) to the second sample, and/or
adding silver sulfate (Ag₂SO₄) to the second sample, and/or
diluting the second sample by calculating a dilution factor, and/or
adding a standard addition concentration to the second sample, and/or
confirming a chloride concentration in the second sample.

9. A system for determining an interference and a concentration of a nitrate, the system comprising:
a cuvette (10) which is configured to have a sample of an aqueous media which comprises the nitrate and a reagent comprising 2,6-dimethylphenol be mixed therein so as to provide a mixed sample (12);
a photometer (14) comprising a light source (16) and a light detector (18), the photometer (14) being configured to determine a first absorbance of the nitrate in the mixed sample colorimetrically at a first wavelength, and to determine a second absorbance of the nitrate in the mixed sample colorimetrically at a second wavelength; and
a computer (20) which comprises a monitor (22) and a database (24), the database (22) comprising comparisons of first absorbance values with respective second absorbance values and ratios of the first absorbance values to the respective second absorbance values, the computer (20) being operatively linked to the photometer (14),
wherein,
the system is configured to perform the method as recited in at least one of claims 1-8.
